# EUROPEAN PATENT APPLICATION

(11) **EP 3 610 752 A1**
(43) Date of publication of application: **19.02.2020**
(21) Application number: 18783768.7
(22) Date of filing: 06.02.2018
(51) Int. Cl.: A41H 42/00, A41D 1/00, A41D 1/06, A61B 5/00, G01L 1/16, D03D 1/00

(54) **CLOTHING-TYPE WEARABLE DEVICE FOR MEASURING MUSCLE ACTIVATION AND MANUFACTURING METHOD THEREFOR**

(30) Priority: 13.04.2017 KR 20170048024
(71) Applicant: Foundation Of Soongsil University Industry Cooperation, Seoul 06978 (KR)
(72) Inventor: KIM, Joo Yong, Seoul 07949 (KR); CHOI, Min Ki, Incheon 21561 (KR)
(74) Representative: Botti, Mario
(86) International application number: PCT/KR2018/001602
(87) International publication number: WO 2018/190502

(57) **Abstract**

A clothing-type wearable device for measuring muscle activation and a manufacturing method therefor are disclosed. The disclosed manufacturing method for the clothing-type wearable device comprises the steps of: (a) forming a clothing pattern on a fabric part; (b) attaching at least one strain sensor generated by means of a conductive solution to at least one location inside the clothing pattern; and (c) sewing the fabric to which the at least one strain sensor has been attached.

## Description

### TECHNICAL FIELD

Embodiments of the present invention relate to a clothing-type wearable device for measuring muscle activity and a method of manufacturing the same.

### BACKGROUND ART

At present, clothing is not only intended to protect a wearer's body, but also intended to be fashionable while expressing the personality and fashion of the wearer's, due to expansion of the concept and function thereof. In addition, functional clothing, i.e. clothing-type wearable devices, having special functions depending on the purpose of use, has been launched to the market. In particular, there is a clothing-type wearing device for measuring muscle activity.

An example of the clothing-type wearing device for measuring muscle activity is a device for performing an electromyogram (EGM). The EGM measuring device measures muscle activity by detecting a micro-current on the muscle surface. However, the EGM measuring device has following drawbacks: EGM measurement needs a sensor for detecting a wide area. An accurate measurement value cannot be obtained at the occurrence of sweat or the contact area is changed, and wearing comfort is not good. In addition, a high conductive material is used to detect a micro-current in the muscle.

### INVENTION

### Technical Problem

Accordingly, the present invention has been made in consideration of the above-described problems occurring in the related art, and the present invention proposes a clothing-type wearable device for measuring muscle activity and a method of manufacturing the same.

Other objects of the present invention will be clearly understood by those skilled in the art from embodiments described hereinafter.

### Technical Solution

According to an embodiment of the present invention, provided is a method of manufacturing a clothing-type wearable device, the method including: a step (a) forming a pattern of clothing on a raw material; a step (b) attaching at least one strain sensor to at least one position within the pattern of clothing, the strain sensor being formed from a conductive solution; a step (c) sewing the raw material to which the at least one strain sensor is attached.

The raw material may include an insulating raw material and at least one electro-conductive string disposed on the insulating raw material, and the at least one strain sensor is connected to one end of each of the at least one electro-conductive string.

The method may further include a step (d) of attaching a communications module for transmitting sensing information, detected by the at least one strain sensor, to an external terminal device, the communications module being connected to the other end of the at least one electro-conductive string.

The step (b) may include: (b1) applying the conductive solution on the at least one position by screen printing; and (b2) forming and attaching the at least one strain sensor by performing heat treatment to the applied conductive solution.

The step (b) may include: (b1) applying the conductive solution on glass; (b2) forming the at least one strain sensor by performing heat treatment to the applied conductive solution; and (b3) detaching the at least one strain sensor from the glass and attaching the detached at least one strain sensor to the at least one position using an adhesive.

The step (b) may include: (b1) submerging the conductive solution into water using a syringe; (b2) forming the at least one strain sensor using the conductive solution submerged in the water; and (b3) attaching the at least one strain sensor to the at least one position by sewing.

The sewing may be performed using cloth or a string, the cloth or the string being formed of the same material as the insulating raw material of the raw material.

A solvent of the conductive solution may be an elastic polymer, and a solute of the conductive solution may be carbon black.

A ratio of the elastic polymer may range from 20% to 40%, and a ratio of the carbon black may range from 60% to 80%.

The clothing may be trousers, and the at least one position may be a point at which a thigh muscle is located.

The at least one strain sensor may detect a degree of muscle expansion of a user on which the clothing-type wearable device is worn.

According to another embodiment of the present invention, provided is a clothing-type wearable device including: a body; a plurality of first open areas and a plurality of second areas connected to the body; at least one strain sensor formed from a conductive solution in at least one position on the body, the first open areas, and the second areas; a communications module transmitting sensing information detected by the at least one strain sensor to an external terminal device; and at least one electro-conductive string disposed on the body, the first open areas, and the second areas, with one end of the at least electro-conductive string being connected to the at least one strain sensor, and the other end of the at least electro-conductive string being connected to the communications module.

### Advantageous Effects

The clothing-type wearable device and the method of manufacturing the same according to the present invention can obtain an accurate measurement value, have superior wearing comfort, and reduce manufacturing costs.

### DESCRIPTION OF DRAWINGS

FIG. 1 is a view illustrating a schematic configuration of a clothing-type wearable device according to an embodiment of the present invention;
FIG. 2 is a view illustrating a flowchart of a printing process of a method of manufacturing the clothing-type wearable device 100 according to the embodiment of the present invention;
FIG. 3 illustrates an example of a pattern of clothing;
FIG. 4 is a view illustrating a flowchart of a bonding process of a method of manufacturing a clothing-type wearable device according to a second embodiment of the present invention;
FIG. 5 illustrates an example of attaching at least one strain sensor to clothing using an adhesive according to an embodiment of the present invention;
FIG. 6 is a view illustrating a flowchart an elastic string process of a method of manufacturing a clothing type wearable device according to a third embodiment of the present invention; and
FIG. 7 illustrates a concept of submerging a conductive solution into water using a syringe according to an embodiment of the present invention.

### MODE FOR INVENTION

Singular forms used herein are intended to include plural forms unless the context clearly indicates otherwise. Terms, such as "include" and "has," used herein should be understood that they are intended to indicate an existence of several components or several steps, disclosed in the specification, and it may also be understood that part of the components or steps may not be included or additional components or steps may further be included. In the following description, terms, such as "unit" and "module," indicate a unit for processing at least one function or operation, wherein the unit and the block may be embodied as hardware or software or embodied by combining hardware and software.

Hereinafter, a variety of embodiments of the present invention will be described in detail with reference to the accompanying drawings.

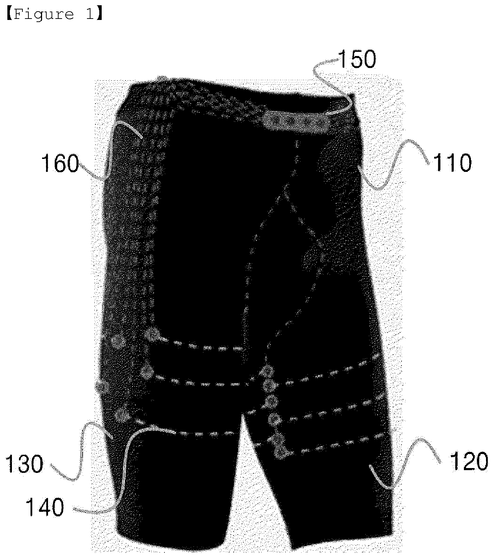

FIG. 1 is a view illustrating a schematic configuration of a clothing-type wearable device according to an embodiment of the present invention.

Referring to FIG. 1, the clothing-type wearable device 100 according to the embodiment of the present invention may be, for example, trouser-type clothing, including a body 110, first open areas 120, second open areas 130, at least one strain sensor 140, a communications module 150, and at least one electro-conductive string 160.

In addition, the clothing-type wearable device 100 may include any type of clothing worn on a body portion of the user, such as long trousers or clothing worn on the upper part of the body. Hereinafter, the clothing-type wearable device 100 may be described by referring to trousers, for the sake of brevity. However, the present invention is not limited thereto.

The body 110 is connected to the first open areas 120 and the second open areas 130, and is worn on the body, i.e. legs, of a user.

The at least one strain sensor 140 or at least one strain gauge is disposed on at least one position on the body 110, the first open areas 120, and the second open areas 130. Here, the strain sensor 140 may be formed from a conductive solution, i.e. a conductive elastic material.

More specifically, the strain sensor 140 is disposed on a deformable object, and serves to measure the deformation and detect the degree of muscle expansion of the user on which the clothing-type wearable device 100 is worn, thereby generating sensing information.

The communications module 150 transmits the sensing information, detected by the at least one strain sensor 140, to an external terminal device.

For example, the position to which the communications module 150 is attached may be the position of the navel. In addition, a textile type touch sensor may be used to control the communications module 150. That is, a capacitor may be comprised of a conductive portion and an insulating portion, so that, when the user touches textile, a touch may be detected in response to a deformation in the textile caused by a pressure or a change in the textile caused by a current flowing to the hand.

The at least one electro-conductive string 160 may be disposed on the body 110, the first open areas 120, and the second open areas 130, and may be formed from a stretchable material. Here, one end of each of the at least one electro-conductive string 160 is connected to the at least one strain sensor 140, and the other end of each of the at least one electro-conductive string 160 is connected to the communications module 150. That is, the at least one electro-conductive string 160 and the communications module 150 are connected to each other via the at least one electro-conductive string 160 corresponding thereto.

Here, a single raw material comprised of an insulating raw material including the electro-conductive string 160 may be manufactured by suitably disposing the at least one electro-conductive string 160 during a fabric or knit manufacturing process for the manufacture of the raw material.

That is, when the user wearing the clothing-type wearable device 100 performs activities, corresponding muscles are expanded. Then, the conductive elastic material (i.e. the strain sensor 140) is expanded by the expanded muscles, and the distances between carbon particles in the conductive elastic material changes, thereby changing the resistance of the conductive elastic material. Afterwards, the communications module 150 allows information regarding the changed resistance to be analyzed using the external terminal device (e.g. a smartphone), so that the muscle activity of the user may be determined. Thus, not only information regarding the muscle activity of the user, but also various pieces of information regarding a muscle mass change, calorie consumption, an amount of exercise, fat percentages of thighs, and the like, may be provided by calculating muscle volume change statistics of the user. The clothing-type wearable device 100 according to the present invention is a textile type device able to respond to muscle expansion in a single texture, and has advantageous features, such as a relatively-inexpensive price compared to those of conventional devices, and wearing comfort due to a thin and light design thereof.

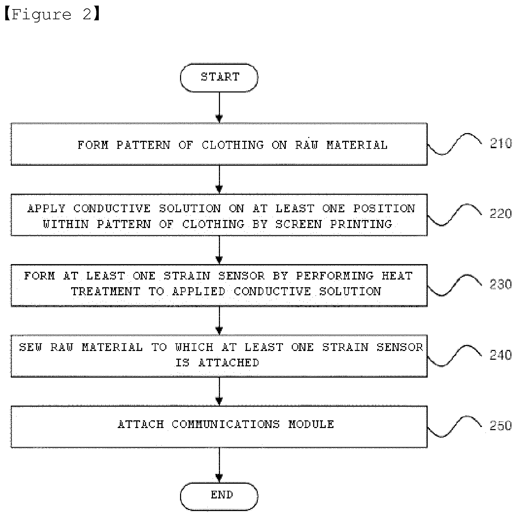

Hereinafter, referring to FIGS. 2 to 7, a method of manufacturing the clothing-type wearable device 100 according to the embodiment of the present invention will be described in detail.

FIG. 2 is a view illustrating a flowchart of a printing process of the method of manufacturing the clothing-type wearable device 100 according to the embodiment of the present invention. Hereinafter, operations of respective steps will be described in detail.

First, in step 210, a pattern of clothing is formed on a raw material.

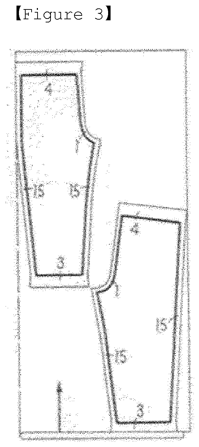

Here, as described above, the raw material may include an insulating raw material and at least one electro-conductive string disposed on the insulating raw material. A single raw material may be manufactured by a single process. In addition, an example of the pattern of clothing is illustrated in FIG. 3.

Afterwards, in step 220, a conductive solution is applied on at least one position within the pattern of clothing (i.e. on the body 110, the first open areas 120, and the second open areas 130) by screen printing.

Here, the screen printing is a process is a printing technique in which a silk screen or another screen (mask) is used to squeeze ink by a manual operation or a photography principle-based method. In the screen printing, meshes of the screen non-corresponding to an image are closed, so that ink is squeezed through meshes of the screen corresponding to the image using a squeegee. Specific descriptions of the screen printing will be omitted, since features of the screen printing are well known to those skilled in the art.

In addition, the conductive material may be a mixture of an elastic polymer and carbon black. The elastic polymer may be a solvent of the conductive solution, while the carbon black may be a solute of the conductive solution. Here, the ratio of the elastic polymer may range from 20% to 40%, while the ratio of the carbon black may range from 60% to 80%. In addition, the conductive solution may be fixed on the raw material using a cover film or a cover raw material.

In addition, the at least one position within the pattern of clothing may be a point at which a thigh muscle is located.

Subsequently, in step 230, at least one strain sensor 140 is formed by performing heat treatment on the applied conductive solution.

That is, in the step 220 and step 230, the at least one strain sensor 140 is formed and attached to the at least one position within the pattern of clothing by directly applying the conductive solution on the at least one position. This may form a thin strain sensor, thereby improving the wearing comfort of the clothing.

Afterwards, in step 240, the raw material to which the at least one strain sensor 140 is attached is sewn. Consequently, a shape of clothing, such as trousers, is completed.

Finally, in step 250, the communications module 150 for transmitting sensing information, detected by the at least one strain sensor 140, to the external terminal device, is attached.

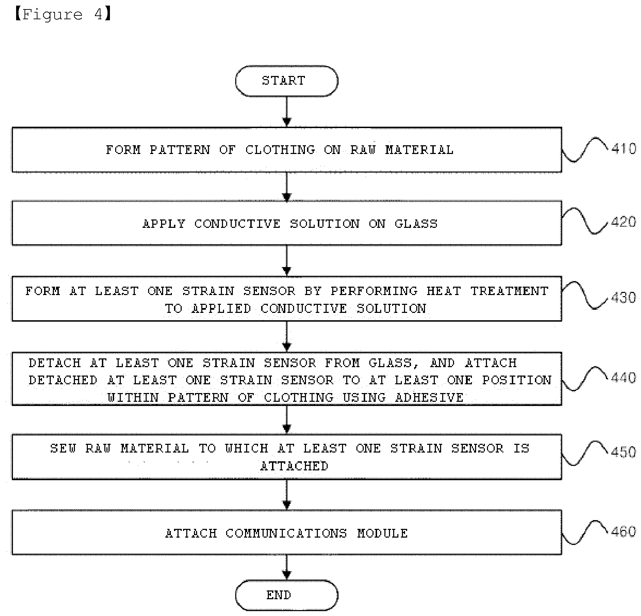

FIG. 4 is a view illustrating a flowchart of a bonding process of a method of manufacturing the clothing-type wearable device 100 according to a second embodiment of the present invention. Hereinafter, operations of respective steps will be described in detail.

First, in step 410, a pattern of clothing is formed on a raw material.

Here, the raw material including an insulating raw material and at least one electro-conductive string may be formed integrally, and the pattern of clothing as illustrated in FIG. 1 may be used.

Afterwards, in step 420, a conductive solution is applied on a piece of glass.

Here, the conductive solution may be applied on the piece of glass by screen printing. In addition, the conductive solution may be a mixture of an elastic polymer and carbon black. Here, the ratio of the elastic polymer may range from 20% to 40%, while the ratio of the carbon black may range from 60% to 80%. In addition, the conductive solution may be fixed on the raw material using a cover film or a cover raw material.

Subsequently, in step 430, the at least one strain sensor 140 is formed by performing heat treatment on the applied conductive solution.

Afterwards, in step 440, the at least one strain sensor 140 is detached from the piece of glass, and the detached at least one strain sensor 140 is attached to at least one position within the pattern of clothing using an adhesive.

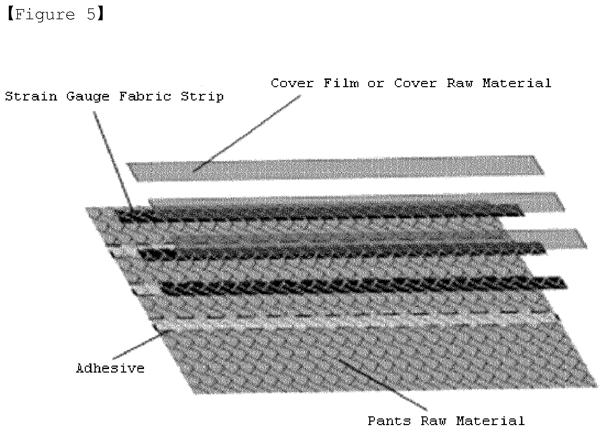

FIG. 5 illustrates a concept of the step 440. The adhesive may be a film type adhesive, and the at least one position within the pattern of clothing may be a point at which the thigh muscle is located.

That is, in the steps 420 to 440, the at least one strain sensor 140 is formed and then is attached to the at least one position within the pattern of clothing using the adhesive. This may form a thin strain sensor, thereby improving the wearing comfort of the clothing.

Afterwards, in step 450, the raw material to which the at least one strain sensor 140 is attached is sewn. Consequently, a shape of clothing, such as trousers, is completed.

Finally, in step 460, the communications module 150 for transmitting sensing information, detected by the at least one strain sensor 140, to the external terminal device, is attached.

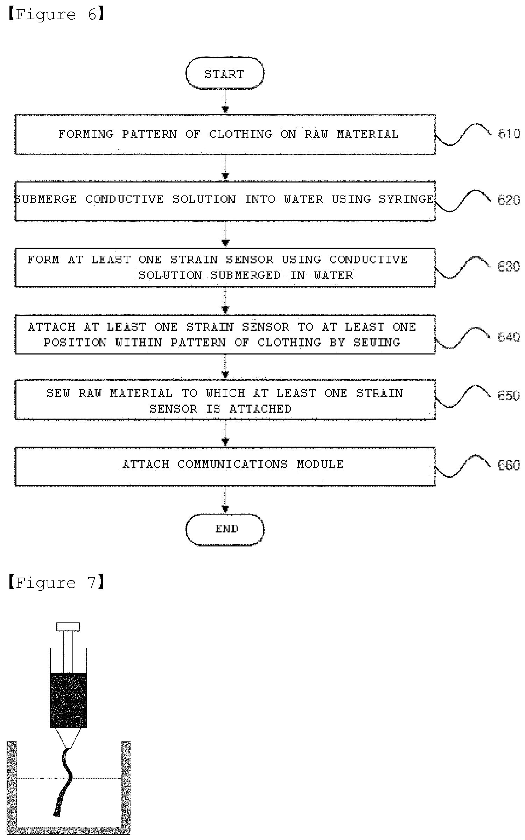

FIG. 6 is a view illustrating a flowchart an elastic string process of the method of manufacturing the clothing-type wearable device 100 according to a third embodiment of the present invention. Hereinafter, operations of respective steps will be described in detail

First, in step 610, a pattern of clothing is formed on a raw material.

Here, the raw material including an insulating raw material and at least one electro-conductive string may be formed integrally, and a pattern of clothing, as illustrated in FIG. 3, may be used.

Afterwards, in step 620, a conductive solution is submerged into water using a dispenser, such as a syringe. An example of this is illustrated in FIG. 7.

Subsequently, in step 630, the at least one strain sensor 140 is formed using the conductive solution submerged in water. That is, the strain sensor 140 is formed by taking the conductive solution out of water.

The strain sensor 140 formed in this case may be thicker than the strain sensor 140 formed by the printing or the bonding. Here, the conductive solution may be applied on a piece of glass by screen printing. In addition, the conductive material may be a mixture of an elastic polymer and carbon black. Here, the ratio of the elastic polymer may range from 20% to 40%, while the ratio of the carbon black may range from 60% to 80%.

Afterwards, in step 640, the at least one strain sensor 140 is attached to at least one position in the pattern of clothing by sewing.

According to an embodiment of the present invention, the sewing is performed using cloth or a string. That is, in a case in which cloth is used, the sewing is performed by placing the strain sensor 140 on the insulating raw material and then putting a piece of cloth over the placed strain sensor 140. In addition, in a case in which a string is used, the strain sensor 140 may be placed on the insulating raw material, and then the placed strain sensor 140 is sewn to the raw material.

Here, the cloth or string may be formed of the same material as the insulating raw material of the raw material. Consequently, the raw material and the at least one strain sensor 140 may be attached by the same process.

Afterwards, in step 650, the raw material to which the at least one strain sensor 140 is attached is sewn. Consequently, a shape of clothing, such as trousers, is completed.

Finally, in step 660, the communications module 150 for transmitting sensing information, detected by the at least one strain sensor 140, to the external terminal device, is attached.

As set forth above, electromyogram-based clothing-type wearable devices of the related art has drawbacks, such as a signal being variable depending on the occurrence of sweat and degradation in wearing comfort due to wide-area electrodes. In contrast, the clothing-type wearable device 100 according to the present invention can accurately measure muscle activity regardless of the skin state of the user or the degree of close contact of the electrodes.

While the present invention has been described above using particular examples, including specific components, by way of limited embodiments and drawings, it is to be appreciated that these are provided merely to aid the overall understanding of the present invention, the present invention is not to be limited to the embodiments above, and various modifications and alterations can be made from the present inventions above by those having ordinary skill in the technical field to which the present invention pertains. Therefore, the spirit of the present invention must not be limited to the embodiments described herein, and the scope of the present invention must be regarded as encompassing not only the claims set forth below, but also their equivalents and variations.

## Claims

1. A method of manufacturing a clothing-type wearable device, the method comprising:
a step (a) forming a pattern of clothing on a raw material;
a step (b) attaching at least one strain sensor to at least one position within the pattern of clothing, the strain sensor being formed from a conductive solution;
a step (c) sewing the raw material to which the at least one strain sensor is attached.

2. The method according to claim 1, wherein the raw material includes an insulating raw material and at least one electro-conductive string disposed on the insulating raw material, and the at least one strain sensor is connected to one end of each of the at least one electro-conductive string.

3. The method according to claim 2, further comprising a step (d) of attaching a communications module for transmitting sensing information, detected by the at least one strain sensor, to an external terminal device, the communications module being connected to the other end of the at least one electro-conductive string.

4. The method according to claim 1, wherein the step (b) comprises:
(b1) applying the conductive solution on the at least one position by screen printing; and
(b2) forming and attaching the at least one strain sensor by performing heat treatment to the applied conductive solution.

5. The method according to claim 1, wherein the step (b) comprises:
(b1) applying the conductive solution on glass;
(b2) forming the at least one strain sensor by performing heat treatment to the applied conductive solution; and
(b3) detaching the at least one strain sensor from the glass and attaching the detached at least one strain sensor to the at least one position using an adhesive.

6. The method according to claim 1, wherein the step (b) comprises:
(b1) submerging the conductive solution into water using a syringe;
(b2) forming the at least one strain sensor using the conductive solution submerged in the water; and
(b3) attaching the at least one strain sensor to the at least one position by sewing.

7. The method according to claim 6, wherein the sewing is performed using cloth or a string, the cloth or the string being formed of the same material as the insulating raw material of the raw material.

8. The method according to claim 1, wherein a solvent of the conductive solution is an elastic polymer, and a solute of the conductive solution is carbon black.

9. The method according to claim 8, wherein a ratio of the elastic polymer ranges from 20% to 40%, and a ratio of the carbon black ranges from 60% to 80%.

10. The method according to claim 1, wherein the clothing is trousers, and the at least one position is a point at which a thigh muscle is located.

11. The method according to claim 1, wherein the at least one strain sensor detects a degree of muscle expansion of a user on which the clothing-type wearable device is worn.

12. A clothing-type wearable device comprising:
a body;
a plurality of first open areas and a plurality of second areas connected to the body;
at least one strain sensor formed from a conductive solution in at least one position on the body, the first open areas, and the second areas;
a communications module transmitting sensing information detected by the at least one strain sensor to an external terminal device; and
at least one electro-conductive string disposed on the body, the first open areas, and the second areas, with one end of the at least electro-conductive string being connected to the at least one strain sensor, and the other end of the at least electro-conductive string being connected to the communications module.

13. The clothing-type wearable device according to claim 12, wherein the at least one strain sensor is formed by applying the conductive solution to the at least one position by screen printing and performing heat treatment to the applied conductive solution.

14. The clothing-type wearable device according to claim 12, wherein the at least one strain sensor is formed by applying the conductive solution on glass and performing heat treatment to the applied conductive solution, the conductive solution being attached to the at least one position using an adhesive.

15. The clothing-type wearable device according to claim 12, wherein the at least one strain sensor is formed from the conductive solution submerged into water using a syringe and is attached to the at least one position by sewing, and
the sewing is performed using cloth or a string, the cloth or the string being formed of the same material as the insulating raw material of the raw material.

16. The clothing-type wearable device according to claim 12, wherein a solvent of the conductive solution is an elastic polymer, and a solute of the conductive solution is carbon black, a ratio of the elastic polymer ranges from 20% to 40%, and a ratio of the carbon black ranges from 60% to 80%.
